(19) Europäisches Patentamt
European Patent Office
Office européen des brevets

(11) **EP 4 269 380 A1**

(12) **EUROPEAN PATENT APPLICATION**
published in accordance with Art. 153(4) EPC

(43) Date of publication:
**01.11.2023 Bulletin 2023/44**

(21) Application number: **21910799.2**

(22) Date of filing: **21.12.2021**

(51) International Patent Classification (IPC):
$C07C\ 41/18$ (2006.01)    $C07C\ 43/17$ (2006.01)
$C07C\ 51/58$ (2006.01)    $C07C\ 59/60$ (2006.01)
$C07C\ 303/22$ (2006.01)    $C07C\ 309/82$ (2006.01)
$C07B\ 61/00$ (2006.01)

(52) Cooperative Patent Classification (CPC):
C07B 61/00; C07C 41/18; C07C 43/17;
C07C 51/58; C07C 59/60; C07C 303/22;
C07C 309/82

(86) International application number:
**PCT/JP2021/047380**

(87) International publication number:
**WO 2022/138659 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.12.2020 JP 2020217715**

(71) Applicant: **AGC INC.**
**Chiyoda-ku,**
**Tokyo 1008405 (JP)**

(72) Inventors:
• **YAMADA, Taku**
**Tokyo 100-8405 (JP)**
• **NIHEI, Toshifumi**
**Tokyo 100-8405 (JP)**

(74) Representative: **Müller-Boré & Partner**
**Patentanwälte PartG mbB**
**Friedenheimer Brücke 21**
**80639 München (DE)**

(54) **METHOD FOR PRODUCING FLUOROVINYL ETHER COMPOUND**

(57)    To provide a method for producing a fluorovinyl ether compound, whereby it is possible to suppress the generation of byproducts.

   The method for producing a fluorovinyl ether compound of the present invention is a method for producing a fluorovinyl ether compound, which comprises heat-treating a compound having a group represented by the formula (1): $F-C(=O)-CF(X)-(CF_2)_n-O-$ in the presence of an oxide containing at least one element selected from the group consisting of alkali metal elements and alkaline earth metal elements, to obtain a fluorovinyl ether compound having a group represented by the formula (2): $CF_2=CF-O-$, wherein the specific surface area of the oxide before the heat treatment is at least 1.0 $m^2/g$. In the formula (1), n is 0 or 1, wherein when n is 0, X is $CF_3$ and when n is 1, X is F.

EP 4 269 380 A1

**Description**

TECHNICAL FIELD

The present invention relates to a method for producing a fluorovinyl ether compound.

BACKGROUND ART

A fluorovinyl ether compound is used, for example, as a monomer for producing a fluorinated polymer. As a method for producing such a fluorovinyl ether compound, Patent Document 1 discloses a method of bringing glass beads (sodium silicate glass) into contact with a perfluoro-2-methoxypropionyl fluoride and heating.

PRIOR ART DOCUMENT

PATENT DOCUMENT

Patent Document 1: U.S. Patent No. 3291843

DISCLOSURE OF INVENTION

TECHNICAL PROBLEM

When the present inventors produced a fluorovinyl ether compound with reference to the production method described in Patent Document 1, they found that the generation of byproducts could not be sufficiently suppressed and that there was room for improvement.
The present invention was made in view of the above problem, and is concerned with providing a method for producing a fluorovinyl ether compound, whereby it is possible to suppress the generation of byproducts.

SOLUTION TO PROBLEM

The present inventors have studied the above problem intensively and, as a result, have found that at the time of heat-treating a compound having group represented by the after-described formula (1) to obtain a compound having a group represented by the after-described formula (2), if an oxide containing at least one element selected from the group consisting of alkali metal elements and alkaline earth metal elements and having a specific surface area of at least $1.0$ $m^2/g$ before the above heat treatment, is used, it is possible to suppress the generation of byproducts, and thus have arrived at the present invention.
That is, the present inventors have found it possible to solve the above problem by the following constructions.

[1] A method for producing a fluorovinyl ether compound, which comprises heat-treating a compound having a group represented by the following formula (1) in the presence of an oxide containing at least one element selected from the group consisting of alkali metal elements and alkaline earth metal elements, to obtain a fluorovinyl ether compound having a group represented by the following formula (2), wherein the specific surface area of the oxide before the heat treatment is at least $1.0$ $m^2/g$:

$$F\text{-}C(=O)\text{-}CF(X)\text{-}(CF_2)_n\text{-}O\text{-} \qquad \text{Formula (1)}$$

$$CF_2=CF\text{-}O\text{-} \qquad \text{Formula (2)}$$

in the formula (1), n is 0 or 1, and when n is 0, X is $CF_3$ and when n is 1, X is F.
[2] The method for producing a fluorovinyl ether compound according to [1], wherein the oxide contains an alkali metal element.
[3] The method for producing a fluorovinyl ether compound according to any one of [1] and [2], wherein the oxide is an oxide containing at least one element selected from the group consisting of alkali metal elements and alkaline earth metal elements, and another metal element.
[4] The method for producing a fluorovinyl ether compound according to any one of [1] to [3], wherein the oxide is a silicate, aluminate, aluminosilicate, borosilicate or aluminoborosilicate containing at least one element selected from the group consisting of alkali metal elements and alkaline earth metal elements.
[5] The method for producing a fluorovinyl ether compound according to any one of [1] to [4], wherein the oxide is an amorphous oxide selected from glass, amorphous silica, amorphous alumina and amorphous silica-alumina, or a crystalline oxide selected from crystalline silica, crystalline alumina and crystalline silica-alumina.

[6] The method for producing a fluorovinyl ether compound according to any one of [1] to [5], wherein the specific surface area of the oxide before the heat treatment is from 1.0 to 700 $m^2/g$.

[7] The method for producing a fluorovinyl ether compound according to any one of [1] to [6], wherein the heat treatment is conducted in the presence of the oxide and another oxide different from the oxide, wherein the amount of the oxide used is from 0.1 to 99 mass% to the total amount of the oxide and another oxide used.

[8] The method for producing a fluorovinyl ether compound according to any one of [1] to [7], wherein the compound having the group represented by the above formula (1) is a perfluorinated compound.

[9] The method for producing a fluorovinyl ether compound according to any one of [1] to [8], wherein the compound represented by the above formula (1) is a compound represented by the following formula (1A):

$$F-C(=O)-CF(CF_3)-O-R^f \qquad \text{Formula (1A)}$$

in the formula (1A), $R^f$ represents a perfluoroalkyl group which may have a monovalent substituent selected from the group consisting of $-C(=O)F$, a sulfonyl fluoride group, a nitrile group and a methyl ester group, or a monovalent group having $-CF_2-$ of the perfluoroalkyl group which may have a monovalent substituent, substituted by an etheric oxygen atom.

[10] The method for producing a fluorovinyl ether compound according to [9], wherein the compound represented by the above formula (1A) is a compound represented by the following formula (1A-1), a compound represented by the following formula (1A-2) or a compound represented by the following formula (1A-3):

$$F-C(=O)-CF(CF_3)-O-Z^{a1} \qquad \text{Formula (1A-1)}$$

($Z^{a1}$ is a perfluoroalkyl group, or a monovalent group having $-CF_2-$ of the perfluoroalkyl group substituted by an etheric oxygen atom),

$$F-C(=O)-CF(CF_3)-O-Q^{a2}-C(=O)-F \qquad \text{Formula (1A-2)}$$

($Q^{a2}$ is a perfluoroalkylene group, or a divalent group having $-CF_2-$ of the perfluoroalkylene group substituted by an etheric oxygen atom),

$$F-C(=O)-CF(CF_3)-O-Q^{a3}(-SO_2F)_q \qquad \text{Formula (1A-3)}$$

($Q^{a3}$ is a (q+1)-valent perfluorohydrocarbon group or a (q+1)-valent group having $-CF_2-$of the perfluorohydrocarbon group substituted by an etheric oxygen atom).

[11] The method for producing a fluorovinyl ether compound according to any one of [1] to [10], wherein the compound represented by the above formula (2) is a compound represented by the following formula (2A):

$$CF_2=CF-O-R^f \qquad \text{Formula (2A)}$$

($R^f$ is synonymous with $R^f$ in the formula (1A)).

[12] The method for producing a fluorovinyl ether compound according to [11], wherein the compound represented by the above formula (2A) is a compound represented by the following formula (2A-1), a compound represented by the following formula (2A-2-1), a compound represented by the following formula (2A-2-2), or a compound represented by the following formula (2A-3):

$$CF_2=CF-O-Z^{a1} \qquad \text{Formula (2A-1)}$$

($Z^{a1}$ in the formula (2A-1) is synonymous with $Z^{a1}$ in the formula (1A-1),

$$CF_2=CF-O-Q^{a2}-C(=O)-F \qquad \text{Formula (2A-2-1)}$$

$$CF_2=CF-O-Q^{a21}-CF=CF_2 \qquad \text{Formula (2A-2-2)}$$

($Q^{a2}$ in the formula (2A-2-1) is synonymous with $Q^{a3}$ in the formula (1A-2), $Q^{a21}$ in the formula (2A-2-2) is a perfluoroalkylene group, or a divalent group having $-CF_2-$ of the perfluoroalkylene group substituted with an etheric oxygen atom),

$$CF_2=CF-O-Q^{a3}(-SO_2F)_q \qquad \text{Formula (2A-3)}$$

($Q^{a3}$ and q in the formula (2A-3) are synonymous with $Q^{a3}$ and q in the formula (1A-3), respectively).

[13] The method for producing a fluorovinyl ether compound according to any one of [1] to [12], wherein the oxide is dried prior to the heat treatment.

ADVANTAGEOUS EFFECTS OF INVENTION

According to the present invention, it is possible to provide a method for producing a fluorovinyl ether compound, whereby it is possible to suppress the generation of byproducts. Further, according to the present invention, it is also possible to provide a method for producing a fluorovinyl ether compound whereby it is possible to suppress the generation of byproducts even when the conversion ratio of raw materials is improved.

DESCRIPTION OF EMBODIMENTS

The meanings of the terms in the present invention are as follows.

A numerical range expressed by using " to " means a range that includes the numerical values listed before and after" to " as the lower and upper limit values.

[Method for producing a fluorovinyl ether compound]

The method for producing a fluorovinyl ether compound of the present invention is a method for producing a fluorovinyl ether compound, which comprises heat-treating a compound having a group represented by the following formula (1) (hereinafter referred to also as "compound 1") in the presence of an oxide containing at least one element selected from the group consisting of alkali metal elements and alkaline earth metal elements to obtain a fluorovinyl ether compound having a group represented by the following formula (2) (hereinafter referred to also as "compound 2"). Further, the specific surface area of the above oxide before the above heat treatment is at least 1.0 m2/g.

Hereinafter, an oxide containing at least one element selected from the group consisting of alkali metal elements and alkaline earth metal elements and having a specific surface area of at least $1.0 \text{ m}^2/\text{g}$ before the above heat treatment will be referred to also as a "specified oxide".

According to the method for producing a fluorovinyl ether compound of the present invention, it is possible to suppress the generation of byproducts. Generally, when the conversion rate of raw materials is improved, the amount of byproducts generated also tends to increase, but, surprisingly, the present inventors have found it possible to suppress the generation of byproducts by using the above specified oxide.

<Compound 1>

Compound 1 is a compound having a group represented by the following formula (1) and is a raw material to be used in the production of compound 2.

$$F-C(=O)-CF(X)-(CF_2)_n-O- \qquad \text{Formula (1)}$$

In the formula (1), n is 0 or 1, and when n is 0, X is $CF_3$ and when n is 1, X is F.

Compound 1 is preferably a perfluorinated compound from the viewpoint of stability of compound 1 for the production of compound 2.

Here, a perfluorinated compound is a compound that contains virtually no hydrogen atoms bonded to carbon atoms and in which all hydrogen atoms bonded to carbon atoms are replaced by fluorine atoms.

The compound represented by the formula (1) is preferably a compound represented by the following formula (1A) (hereinafter referred to also as "compound 1A") from the viewpoint of the stability of compound 1 for producing compound 2.

$$F-C(=O)-CF(CF_3)-O-R^f \qquad \text{Formula (1A)}$$

$R^f$ is a perfluoroalkyl group which may have a monovalent substituent selected from the group consisting of -C(=O)F, a sulfonyl fluoride group (-SO_2F), a nitrile group (-CN) and a methyl ester group (-C(O)OCH_3), or a monovalent group having -CF_2- of the perfluoroalkyl group which may have the above monovalent substituent substituted by an etheric oxygen atom.

The number of carbon atoms in the perfluoroalkyl group in $R^f$ is preferably from 1 to 20, more preferably from 1 to 15, particularly preferably from 1 to 10, from such a viewpoint that it is possible to suppress an increase of the boiling point due to the increase in the number of carbon atoms, and the production of compound 2 will be easier.

The perfluoroalkyl group in $R^f$ may be linear or branched.

If the perfluoroalkyl group in $R^f$ has a monovalent substituent, the number of monovalent substituents may be one, two or more. The monovalent substituent may be bonded to any carbon atom.

Among the monovalent substituents, -C(=O)F and a sulfonyl fluoride group ($-SO_2F$) are preferred.

The number of etheric oxygen atoms which the monovalent group in $R^f$ has, may be one, two or more. The etheric oxygen atom is preferably located between the carbon-carbon atomic bonds of the perfluoroalkyl group.

Compound 1A is, from the viewpoint that it facilitates the production of compound 2, preferably a compound represented by the following formula 1A-1 (hereinafter referred to also as "compound 1A-1"), a compound represented by the following formula 1A-2 (hereinafter referred to also as "compound 1A-2"), or a compound represented by the following formula 1A-3 (hereinafter referred to also as "compound 1A-3").

$$F\text{-}C(=O)\text{-}CF(CF_3)\text{-}O\text{-}Z^{a1} \qquad \text{Formula (1A-1)}$$

$Z^{a1}$ is a perfluoroalkyl group, or a monovalent group having $-CF_2-$ of the perfluoroalkyl group substituted by an etheric oxygen atom.

The number of carbon atoms in the perfluoroalkyl group is preferably from 1 to 10, particularly preferably from 1 to 6. The perfluoroalkyl group may be linear or branched.

The number of etheric oxygen atoms which the monovalent group in $Z^{a1}$ has, may be one, two or more. The etheric oxygen atom is preferably located between the carbon-carbon bonds of the perfluoroalkyl group.

Compound 1A-1 is preferably a compound represented by the following formula 1A-1-1 (hereinafter referred to also as "compound 1A-1-1") or a compound represented by the following formula 1A-1-2 (hereinafter referred to also as "compound 1A-1-2").

$$F\text{-}C(=O)\text{-}CF(CF_3)\text{-}O\text{-}R^{f11} \qquad \text{Formula (1A-1-1)}$$

$R^{f11}$ is a perfluoroalkyl group. The number of carbon atoms in the perfluoroalkyl group is preferably from 1 to 10, particularly preferably from 1 to 6. The perfluoroalkyl group may be linear or branched.

$$F\text{-}C(=O)\text{-}CF(CF_3)\text{-}O\text{-}(R^{f12}O)_{m1}\text{-}R^{f13} \qquad \text{Formula (1A-1-2)}$$

$R^{f12}$ is a perfluoroalkylene group. The number of carbon atoms in the perfluoroalkylene group is preferably from 1 to 10, particularly preferably from 1 to 6. The perfluoroalkylene group may be linear or branched.

$R^{f13}$ is a perfluoroalkyl group. The number of carbon atoms in the perfluoroalkyl group is preferably from 1 to 10, particularly preferably from 1 to 6. The perfluoroalkyl group may be linear or branched.

m1 is an integer of at least 1, preferably from 1 to 6, particularly preferably from 1 to 3. In a case where m1 is at least 2, the plurality of ($R^{f12}O$) may be the same or different from each other.

$$F\text{-}C(=O)\text{-}CF(CF_3)\text{-}O\text{-}Q^{a2}\text{-}C(=O)\text{-}F \qquad \text{Formula (1A-2)}$$

The definitions of the respective groups in the formula (1A-2) are as follows.

$Q^{a2}$ is a perfluoroalkylene group, or a divalent group having $-CF_2-$ of the perfluoroalkylene group substituted by an etheric oxygen atom.

The number of carbon atoms in the perfluoroalkylene group is preferably from 1 to 10, particularly preferably from 1 to 6. The perfluoroalkylene group may be linear or branched.

The number of etheric oxygen atoms in the divalent group in $Q^{a2}$ may be one, two or more. The etheric oxygen atom is preferably located between the carbon-carbon bonds of the perfluoroalkylene group.

Compound 1A-2 is preferably a compound represented by the following formula 1A-2-1 (hereinafter referred to also as "compound 1A-2-1") or a compound represented by the following formula 1A-2-2 (hereinafter referred to also as "compound 1A-2-2").

$$F\text{-}C(=O)\text{-}CF(CF_3)\text{-}O\text{-}R^{f21}\text{-}C(=O)\text{-}F \qquad \text{Formula (1A-2-1)}$$

$R^{f21}$ is a perfluoroalkylene group. The number of carbon atoms in the perfluoroalkylene group is preferably from 1 to 10, particularly preferably from 1 to 6. The perfluoroalkylene group may be linear or branched.

$$F\text{-}C(=O)\text{-}CF(CF_3)\text{-}O\text{-}(R^{f22}O)_{m2}\text{-}R^{f23}\text{-}C(=O)\text{-}F \qquad \text{Formula (1A-2-2)}$$

$R^{f22}$ is a perfluoroalkylene group. The number of carbon atoms in the perfluoroalkylene group is preferably from 1 to 10, particularly preferably from 1 to 6. The perfluoroalkylene group may be linear or branched.

$R^{f23}$ is a perfluoroalkylene group. The number of carbon atoms in the perfluoroalkylene group is preferably from 1 to 8, particularly preferably from 1 to 5. The perfluoroalkylene group may be linear or branched.

m2 is an integer of at least 1, preferably from 1 to 6, particularly preferably from 1 to 3. In a case where m2 is at least 2, the plurality of ($R^{f22}O$) may be the same or different from each other.

$$F\text{-}C(=O)\text{-}CF(CF_3)\text{-}O\text{-}Q^{a3}(\text{-}SO_2F)_q \qquad \text{Formula (1A-3)}$$

The definitions of the respective groups in the formula (1A-3) are as follows.

$Q^{a3}$ is a (q+1)-valent perfluorohydrocarbon group, or a (q+1)-valent group having $-CF_2-$ of the perfluorohydrocarbon group substituted by an etheric oxygen atom.

The number of carbon atoms in the perfluorohydrocarbon group is preferably from 1 to 10. The perfluorohydrocarbon group may be linear or branched.

The number of etheric oxygen atoms in the (q+1)-valent group in $Q^{a3}$ may be one, two or more. The etheric oxygen atom is preferably located between the carbon-carbon atomic bonds of the perfluorohydrocarbon group.

q is an integer of from 1 to 3.

Compound 1A-3 is preferably a compound represented by the following formula 1A-3-1 (hereinafter referred to also as "compound 1A-3-1") or a compound represented by the following formula 1A-3-2 (hereinafter referred to also as "compound 1A-3-2").

$$F\text{-}C(=O)\text{-}CF(CF_3)\text{-}(OCF_2CFZ^{a1})_{m3}\text{-}(O)_{p3}\text{-}(CF_2)_{n3}\text{-}SO_2F \qquad \text{Formula (1A-3-1)}$$

The definitions of the respective groups in the formula (1A-3-1) are as follows.

$Z^{a1}$ is a fluorine atom or a trifluoromethyl group.
m3 is an integer of from 0 to 3.
p3 is 0 or 1.
n3 is an integer of from 1 to 12.
However, m3 + p3 is at least 1.

$$F\text{-}C(=O)\text{-}CF(CF_3)\text{-}O\text{-}(CF_2)_{n4}\text{-}(O)_{p4}\text{-}C(Z^{a2})(\text{-}Q^{a31}\text{-}SO_2F)(\text{-}Q^{a32}\text{-}SO_2F) \qquad \text{Formula (1A- 3-2)}$$

The definitions of the respective groups in the formula (1A-3-2) are as follows.

$Z^{a2}$ is a perfluoroalkyl group, a monovalent group having $-CF_2-$ of the perfluoroalkyl group substituted by an etheric oxygen atom, or a fluorine atom.

The number of carbon atoms in the perfluoroalkyl group is preferably from 1 to 6. The perfluoroalkyl group may be linear or branched, but linear is preferred.

The number of etheric oxygen atoms in the monovalent group in $Z^{a2}$ may be one, two or more. Further, the etheric oxygen atom may be located between the carbon-carbon atomic bonds of the perfluoroalkyl group, or may be located at the carbon atom bond terminal and on the side directly bonded to C (carbon atom) in $-C(Z^{a2})$.

n4 is an integer of from 1 to 3.
p4 is 0 or 1.

$Q^{a31}$ is a perfluoroalkylene group, or a divalent group having $-CF_2-$ of the perfluoroalkylene group substituted by an etheric oxygen atom.

$Q^{a32}$ is a single bond, a perfluoroalkylene group, or a divalent group having $-CF_2-$ of the perfluoroalkylene group substituted by an etheric oxygen atom.

The number of carbon atoms in the perfluoroalkylene group is preferably from 1 to 6, particularly preferably from 1 to 4. The perfluoroalkylene group may be linear or branched, but linear is preferred.

The number of etheric oxygen atoms which the divalent group in $Q^{a31}$ and $Q^{a32}$ has, may be one, two or more. Further, the etheric oxygen atom is preferably located between the carbon-carbon bonds of the perfluoroalkylene group, or at the carbon atom bond terminal and on the side directly bonded to C (carbon atom) in $-C(Z^{a2})$.

<Specified oxide>

The specified oxide contains at least one element selected from the group consisting of alkali metal elements and alkaline earth metal elements (hereinafter referred to also as a "specified element"). It is preferred that the specified oxide contains an alkali metal element, because the generation of byproducts can thereby be more suppressed.

As specific examples of alkali metal elements, lithium, sodium, potassium, rubidium, cesium and francium, may be mentioned, and sodium and potassium are preferred because they can better suppress the generation of byproducts. Only one type or two or more types of alkali metal elements may be contained.

As specific examples of alkaline earth metal elements, beryllium, magnesium, calcium, strontium and barium may be mentioned, and magnesium, calcium and barium are preferred because they can better suppress the generation of byproducts. Only one type or two or more types of alkaline earth metal elements may be contained.

Further, components that can be contained in the specified oxide, may be known metallic elements or compounds that can be converted from F-C(=O)- to carboxylates or alkyl ethers that induce fluorovinyl ether compounds, as shown in the known literature (J. Am. Chem. Soc, 1953, 75, 18, 4525-4528), etc., and, for example, silver, ammonium, hydroxides, and alkoxides may be contained.

From the viewpoint of producing compound 2, the specified element may be present as an oxide or as an ionic species.

It is preferred that the specified oxide contains other elements other than the specified element as elements that play a role in supporting the specified element as an oxide or ionic species. Specific examples of such other elements may be silicon, aluminum, manganese, lead, boron, zinc, zirconium, phosphorus and magnesium. From the viewpoint that the generation of byproducts can be more suppressed, silicon and aluminum are preferred, and these elements are present as oxides.

Depending on the ratio of the specified element to other elements, the valence of other elements will vary, but the valence is not limited from the viewpoint of its role in supporting the specified element as an oxide or ionic species or in producing compound 2.

The specified oxide may be a single oxide of the specified element or an oxide containing the specified metal element and other metal elements, but it is preferably an oxide containing the specified metal element and other metal elements. The oxide containing the specified metal element and other metal elements may be a composite oxide containing an oxide of the specified element and an oxide of other elements.

The specified oxide is preferably a silicate, an aluminate, an aluminosilicate, a borosilicate or an aluminoborosilicate containing a specified element.

The structure of the specified oxide may be amorphous or crystalline, and may be non-porous or porous.

Specific examples of the amorphous specified oxide may be various types of glass, amorphous silica (also called non-crystalline silica, e.g. silica gel), amorphous alumina (e.g. alumina gel), and amorphous silica-alumina. Further, specific examples of the crystalline specified oxide may be crystalline silica (e.g. cristobalite, mesoporous silica, silicalite), crystalline alumina (e.g. $\gamma$-alumina, $\eta$-alumina, $\theta$-alumina, $\alpha$-alumina, etc.), crystalline silica-alumina (those with non-ordered structure: silica-alumina, etc.; those with ordered structure: mesoporous silica-alumina, zeolite, etc.).

As the glass, a known composition having $SiO_2$ as the main component may be used, and it is preferred to contain at least one component selected from $Li_2O$, $Na_2O$, $K_2O$, MgO, CaO and BaO as a specified element in an amount of from 0.01 to 20 mass%.

It is preferred that the glass contains at least one of sodium and potassium, as it is possible to better suppress the generation of byproducts.

As examples of the specific composition, soda-lime glass containing $SiO_2$, $Na_2O$ and CaO as the main components; potassium crystal glass containing $SiO_2$, $Na_2O$, $K_2O$ and CaO as the main components; aluminosilicate glass containing $SiO_2$, $Al_2O_3$, $Na_2O$ and $K_2O$ as the main components; lead glass containing $SiO_2$, $K_2O$ and PbO as the main components; borosilicate glass containing $SiO_2$, $B_2O_3$, $Na_2O$ and $K_2O$ as the main components; aluminoborosilicate glass containing $SiO_2$, $Na_2O$, $K_2O$, $Al_2O_3$ and $B_2O_3$ as the main components, may be mentioned, but any oxide composition that contains a specified metal element and other metal elements is acceptable without being limited to these.

Si/Al (molar ratio of silica element to aluminum element) in the glass is preferably at most 100 from the viewpoint of increasing alkali content, and at least 0.9 from the viewpoint of acid resistance.

At the time of applying surface roughening treatment with acid gas, etc., the average pore size of the uneven structure to be formed on the glass surface is preferably at most 30 nm from the viewpoint of better suppression of generation of byproducts, and preferably at least 5 nm from the viewpoint of improving the reactivity.

As amorphous silica, it is possible to use silica gel made from sodium metasilicate ($Na_2SiO_3$) as raw material, or precipitation method silica or gel method silica obtainable by neutralization reaction of sodium silicate ($Na_2O \cdot nSiO_2 \cdot mH_2O$) with an acid, and it is preferred to contain, as the specified element, at least one oxide selected from $Li_2O$, $Na_2O$, $K_2O$, MgO, CaO and BaO in an amount of from 0.01 to 20 mass%.

The amorphous silica preferably contains at least one of sodium and potassium, as it is thereby possible to better suppress the generation of byproducts.

The average pore size of the amorphous silica is preferably at most 30 nm from the viewpoint of better suppression of the generation of byproducts, and preferably at least 5 nm from the viewpoint of improving the reactivity.

As the amorphous alumina, it is possible to use alumina gel ($Al_2O_3 \cdot nH_2O$) or an aluminate having aluminum or aluminum hydroxide dissolved in alkali hydroxide solution, and it is preferred to contain, as the specified element, at least one component selected from $Li_2O$, $Na_2O$, $K_2O$, MgO, CaO and BaO in an amount of from 0.01 to 20 mass%.

The amorphous alumina preferably contains at least one of sodium and potassium, as it is thereby possible to better suppress the generation of byproducts.

As examples of specific compositions, an alkali metal salt taking the structural formula of $MAlO_2$ (M is a monovalent metal) such as $NaAlO_2$ or $KAlO_2$, a compound taking the structural formula of $MAlO_2 \cdot nH_2O$ such as $NaAlO_2 \cdot 5/4H_2O$, $NaAlO_2 \cdot 3H_2O$ or $KAlO_2 \cdot 3/2H_2O$, and a compound taking the structural formula of $xM_2O \cdot yAl_2O_2 \cdot zH_2O$ (including z=0) as a general formula, may be mentioned.

The average pore size of the amorphous alumina is preferably at most 30 nm from the viewpoint of better suppression of generation of byproducts, and preferably at least 5 nm from the viewpoint of improving the reactivity.

As amorphous silica-alumina, it is possible to use, for example, silica-alumina obtained by calcining silica-alumina gel prepared by the sol-gel method, and it is preferred to contain, as a specified element, at least one component selected from $Li_2O$, $Na_2O$, $K_2O$, MgO, CaO and BaO in an amount of from 0.01 to 20 mass%.

The amorphous silica-alumina preferably contains at least one of sodium and potassium, as it is thereby possible to better suppress the generation of byproducts.

From the viewpoint of increasing the alkali content, Si/Al in the amorphous silica-alumina is preferably at most 100, and from the viewpoint of acid resistance, Si/Al in the amorphous silica-alumina is at least 0.9.

The average pore size of the amorphous silica-alumina is preferably at most 30 nm from the viewpoint of better suppressing the generation of byproducts, and preferably at least 5 nm from the viewpoint of improving the reactivity.

As crystalline silica, it is possible to use quartz, cristobalite, keatite, stishovite, etc., and it is preferred to contain, as a specified element, at least one component selected from $Li_2O$, $Na_2O$, $K_2O$, MgO, CaO and BaO in an amount of from 0.01 to 20 mass%.

The crystalline silica preferably contains at least one of sodium and potassium, as it is thereby possible to better suppress the generation of byproducts.

The average pore size of the amorphous silica-alumina is preferably at most 30 nm from the viewpoint of better suppressing the generation of byproducts, and preferably at least 5 nm from the viewpoint of improving the reactivity.

As crystalline alumina, it is possible to use $\gamma$-$Al_2O_3$, $\eta$-$Al_2O_3$, $\theta$-$Al_2O_3$, $\alpha$-$Al_2O_3$, etc., and it is preferred to contain, as a specified element, at least one component selected from $Li_2O$, $Na_2O$, $K_2O$, MgO, CaO and BaO in an amount of from 0.01 to 20 mass%.

Further, as the crystalline alumina, a spinel structure or defect spinel structure that forms as a sintered product between a monovalent basic oxide ($Na_2O$ or $K_2O$) or a divalent weakly basic oxide (MgO, CoO, NiO, CuO, ZnO or MnO) and $Al_2O_3$, may be mentioned.

The crystalline alumina preferably contains at least one of sodium and potassium, as it is thereby possible to better suppress the generation of byproducts.

The average pore size of the crystalline alumina is preferably at most 30 nm from the viewpoint of better suppression of the generation of byproducts, and preferably at least 5 nm from the viewpoint of improving the reactivity.

As crystalline silica-alumina with non-ordered structure, it is possible to use mullite or kaolinite of aluminosilicate having a single chain structure, and it is preferred to contain, as a specified element, at least one component selected from $Li_2O$, $Na_2O$, $K_2O$, MgO, CaO and BaO in an amount of from 0.01 to 20 mass%.

The crystalline silica-alumina with non-ordered structure preferably contains at least one of sodium and potassium.

Si/Al in the crystalline silica-alumina with non-ordered structure is preferably at most 100 from the viewpoint of increasing alkali content, and preferably at least 0.9 from the viewpoint of acid resistance.

The average pore size of the crystalline silica-alumina with non-ordered structure is preferably at most 30 nm from the viewpoint of better suppression of the generation of byproducts, and preferably at least 5 nm from the viewpoint of improving the reactivity.

As crystalline silica-alumina with ordered structure, it is possible to use mesoporous silica-alumina or zeolite, and it is preferred to contain as a specified element, at least one component selected from $Li_2O$, $Na_2O$, $K_2O$, MgO, CaO and BaO in an amount of from 0.01 to 20 mass%.

The crystalline silica-alumina with ordered structure preferably contains at least one of sodium and potassium from the viewpoint of better suppressing the generation of byproducts.

Si/Al in the crystalline silica-alumina with ordered structure is, from the viewpoint of increasing the alkali content, preferably at most 100 and, from the viewpoint of acid resistance, preferably at least 0.9, more preferably at least 3.0, further preferably at least 5.0.

The average pore size of the crystalline silica-alumina with ordered structure is preferably at most 30 nm from the viewpoint of better suppressing the generation of byproducts, and preferably at least 5 nm from the viewpoint of improving the reactivity.

As the crystal structures of zeolite, known crystal structures can be used, and a list of them is disclosed by the International Zeolite Association (http://www.iza-structure.org/). Examples of industrially applicable structures include A-type, ferrierite, mordenite, L-type, X-type, Y-type, CHA-type, TON-type, AFI-type, BEA-type, CON-type, MTW-type, CFI-type, and MEL-type.

The zeolite preferably contains at least one of sodium and potassium, as it is thereby possible to better suppress the generation of byproducts.

Si/Al in the zeolite is, from the viewpoint of increasing the amount of cationic species (such as alkali metals) present to compensate for the negative charge of the tetracoordinated Al sites, preferably at most 100, and, from the viewpoint of acid resistance, preferably at least 0.9, more preferably at least 3.0, further preferably at least 5.0.

As the ring structure of the zeolite structure, from the viewpoint of better suppressing the generation of byproducts, the LTA, UFI, AEI, CHA, AFX, LEV, DDR and RHO types having a 8-membered ring with a small pore size, are preferred; the LTA, CHA and DDR types are more preferred; and from the viewpoint of improving the reactivity, the BEA, CFI, AFI, FAU, LTL, MTW, MOR and FER types having a 12- or 14-membered ring with a large pore size, are preferred, and the BEA, FAU, LTL, MOR and FER types, are more preferred.

The zeolite containing a specified element may be natural or synthetic. The zeolite containing a specified element may be commercially available and may be in a powder or molded form.

The specific surface area of the specified oxide before the heat treatment as described later is at least 1.0 $m^2/g$, and from the viewpoint of better suppressing the generation of byproducts, preferably at least 2 $m^2/g$, more preferably at least 10 $m^2/g$, particularly preferably at least 20 $m^2/g$.

Of the specified oxide, from the viewpoint of the structural stability during the heat treatment as described later, the specific surface area before the heat treatment as described later, is preferably at most 1,000 $m^2/g$, more preferably at most 800 $m^2/g$, particularly preferably at most 700 $m^2/g$.

The specific surface area of the specified oxide is the value obtained by analyzing measurement results by the BET method with a device whose measurement principle is a gas adsorption method using nitrogen gas (e.g. "3Flex" manufactured by Micromeritics Instrument Corporation). In a case where the specified oxide is a porous material or an aggregate of particles, the specific surface area includes the total surface area of the structure including the inside of pores and pore spaces, as well as the external surface area excluding the inside of pores and pore spaces. A known analytical method may be used to calculate the external surface area, and, for example, the t-plot method may be used to analyze and determine the external surface area. Further, when the measurement results are analyzed by the BJH method, the pore size distribution and average pore size of the specified oxide can also be obtained.

Here, among oxides containing specified elements, an oxide that does not meet the above specific surface area in the untreated state, may be made to have the above specific surface area by applying heat treatment, surface roughening treatment, etc.

The heat treatment is carried out, for example, by heating in an electric furnace or a reaction tube in an oxygen-containing gas such as air or under an inert gas atmosphere such as nitrogen. The heat treatment temperature varies depending on the crystal structure and heat resistance of the oxide, but from the viewpoint of improving the specific surface area, at most 1,000°C is preferred, at most 800°C is more preferred, and at most 600°C is particularly preferred.

The surface roughening treatment is carried out, for example, by contact or immersion in an acid or alkaline solution or by contact with an acidic gas.

The specified oxide is preferably crystalline from the viewpoint of structural control and reproducibility at the time of its production.

In the present invention, in the diffraction pattern measured by XRD (X-ray diffraction instrument, for example, Smart Lab manufactured by Rigaku Corporation), those containing diffraction peaks corresponding to a crystalline structure as the main component are considered to be crystalline, and those containing no diffraction peaks corresponding to a crystalline structure as the main component, are considered to be amorphous.

The specified oxide may be in a powder or granular form, and may take a fluidized bed or fixed bed reaction format, depending on the particle size.

<Other oxides>

The heat treatment as described later may be conducted in the presence of the specified oxide and other oxides different from the specified oxide.

Other oxides may be oxides that do not contain a specified element and oxides with a specific surface area of less than 1.0 $m^2/g$.

Specific examples of oxides that do not contain a specified element may be zinc oxide, silica, alumina, zirconia and titania.

A specific example of the oxide with a specific surface area of less than 1.0 m$^2$/g may be silicate glass containing SiO$_2$ that has not been surface-roughened, as the main component.

<Production process>

The method for producing a fluorovinyl ether compound of the present invention, has a heating step of heat-treating compound 1 in the presence of a specified oxide.

This causes a thermal decomposition reaction of compound 1, whereby compound 2 is obtainable. Specifically, after an intermediate is formed by the reaction of compound 1 with the specified oxide, thermal decomposition and decarboxylation of the intermediate occur, whereby compound 2 is obtainable.

The lower limit of the reaction temperature is, from the viewpoint of the reactivity of compound 1, preferably at least 150°C, more preferably at least 180°C, particularly preferably at least 200°C.

The upper limit of the reaction temperature is, from the viewpoint of better suppressing of the generation of byproducts, preferably at most 380°C, more preferably at most 360°C, particularly preferably at most 350°C, most preferably at most 310°C. It is preferred to lower the reaction temperature from the viewpoint of reducing the heating energy.

The reaction time is not particularly limited, but is preferably from 0.1 to 120 seconds, particularly preferably from 0.5 to 60 seconds.

Here, the reaction time means the contact time between the specified oxide and compound 1. For example, when a tube-type reactor is to be used, the contact time can be calculated from the amount of gas containing compound 1 to be circulated in the tube-type reactor and the value of the filling volume of the specified oxide in the tube-type reactor.

The reaction pressure is not particularly limited, but is preferably from 0 to 1 MPaG, particularly preferably from 0 to 0.1 MPaG.

The reaction of compound 1 in the heating step may be a gas phase reaction, a liquid phase reaction, or a solid phase reaction, but is preferably a gas phase reaction from the viewpoint that the production of compound 2 will be thereby easy.

In a case where the reaction of compound 1 is a gas phase reaction, it is preferred to use compound 1 as diluted with an inert gas or the like. Specific examples of the inert gas may be nitrogen gas, carbon dioxide gas, helium gas and argon gas.

In a case where an inert gas is to be used, the amount of the inert gas to be used, is preferably from 50 to 99.9 mol%, particularly preferably from 80 to 99.9 mol%, to the total of the amounts of compound 1 and the inert gas.

The specified oxide may be used in a fixed bed method or in a fluidized bed method.

The amount of the specified oxide to be used, is preferably from 0.00001 to 10 kg, particularly preferably from 0.005 to 5 kg, to 1 mol of compound 1.

In a case where the specified oxide is to be used in combination with other oxides, the amount of the specified oxide to be used, is preferably from 0.1 to 99 mass%, more preferably from 0.1 to 80 mass%, particularly preferably from 0.1 to 70 mass%, to the total of the amounts of the specified oxide and other oxides to be used, from the viewpoint that the generation of byproducts can be better suppressed.

The method for producing a fluorovinyl ether compound of the present invention preferably has a drying step of dry treating the specified oxide prior to the heat treatment. In particular, in the presence of water, drying step can further suppress the generation of byproducts that are difficult to separate.

The method for drying treatment of the specified oxide is not particularly limited, but may, for example, be a method of heating the specified oxide.

When the drying treatment is conducted by heating, the heating temperature is preferably from 100 to 450°C, particularly preferably from 150 to 450°C. Further, when the drying treatment is conducted by heating, the heating time is preferably from 10 minutes to 1 week, particularly preferably from 30 minutes to 24 hours.

<Compound 2>

The fluorovinyl ether compound obtainable by the production method in the present invention is a compound having a group represented by the following formula (2) (i.e. compound 2).

$$CF_2=CF-O-\qquad \text{Formula (2)}$$

In the production method in the present invention, when compound 1A is used, a compound represented by the following formula (2A) is obtainable.

$$CF_2=CF\text{-}O\text{-}R^f \qquad \text{Formula (2A)}$$

$R^f$ in the formula (2A) is synonymous with $R^f$ in the formula (1A).

In the production method in the present invention, when compound 1A-1 is used, the compound represented by the following formula (2A-1) is obtainable.

$$CF_2=CF\text{-}O\text{-}Z^{a1} \qquad \text{Formula (2A-1)}$$

$Z^{a1}$ in the formula (2A-1) is synonymous with $Z^{a1}$ in the formula (1A-1).

In the production method in the present invention, when compound 1A-1-1 is used, the compound represented by the following formula (2A-1-1) is obtainable.

$$CF_2=CF\text{-}O\text{-}R^{f11} \qquad \text{Formula (2A-1-1)}$$

$R^{f11}$ in the formula (2A-1-1) is synonymous with $R^{f11}$ in the formula (1A-1-1).

As specific examples of the compound represented by the formula (2A-1-1), $CF_2=CF\text{-}O\text{-}CF_3$, $CF_2=CF\text{-}O\text{-}CF_2CF_3$ and $CF_2=CF\text{-}O\text{-}CF_2CF_2CF_3$ may be mentioned.

In the production method in the present invention, when compound 1A-1-2 is used, the compound represented by the following formula (2A-1-2) is obtainable.

$$CF_2=CF\text{-}O\text{-}(R^{f12}O)_{m1}\text{-}R^{f13} \qquad \text{Formula (2A-1-2)}$$

$R^{f12}$, $R^{f13}$ and m1 in the formula (2A-1-2) are synonymous with $R^{f12}$, $R^{f13}$ and m1 in the formula (1A-1-2), respectively.

As specific examples of the compound represented by the formula (2A-1-2), $CF_2=CF\text{-}O\text{-}CF_2\text{-}OCF_2CF_3$, $CF_2=CF\text{-}O\text{-}CF_2O\text{-}CF_3$, $CF_2=CF\text{-}O\text{-}CF_2O\text{-}CF_2CF_2O\text{-}CF_3$, $CF_2=CF\text{-}O\text{-}CF_2CF_2CF_2O\text{-}CF_3$, $CF_2=CF\text{-}O\text{-}CF_2CF(CF_3)O\text{-}CF_2CF_2CF_3$ and $CF_2=CF\text{-}O\text{-}CF_2CFO\text{-}CF_2CFO\text{-}CF_2CF_3$ may be mentioned.

In the production method in the present invention, when compound 1A-2 is used, a compound represented by the following formula (2A-2-1) or a compound represented by the following formula (2A-2-2) is obtainable by suitably adjusting the production conditions. However, in order to obtain a compound represented by the formula (2A-2-2), $Q^{a2}$ in the above formula (1A-2) is required to have $-CF(CF_3)-$ or $-CF_2\text{-}CF_2-$ directly bonded to $-C(=O)\text{-}F$.

$$CF_2=CF\text{-}O\text{-}Q^{a2}\text{-}C(=O)\text{-}F \qquad \text{Formula (2A-2-1)}$$

$$CF_2=CF\text{-}O\text{-}Q^{a21}\text{-}CF=CF_2 \qquad \text{Formula (2A-2-2)}$$

$Q^{a2}$ in the formula (2A-2-1) is synonymous with $Q^{a3}$ in the formula (1A-2).

$Q^{a21}$ in the formula (2A-2-2) is a perfluoroalkylene group, or a divalent group having $-CF_2-$ of a perfluoroalkylene group substituted by an etheric oxygen atom. The number of carbon atoms in the perfluoroalkylene group is preferably from 1 to 8, particularly preferably from 1 to 4. The perfluoroalkylene group may be linear or branched. The number of etheric oxygen atoms which the divalent group in $Q^{a21}$ has, may be one, two or more. The etheric oxygen atom is preferably located between the carbon-carbon bonds of the perfluoroalkylene group.

In the production method in the present invention, when compound 1A-2-1 is used, a compound represented by the following formula (2A-2-11) or a compound represented by the following formula (2A-2-12) is obtainable. However, in order to obtain the compound represented by the formula (2A-2-12), $R^{f21}$ in the above formula (1A-2-1) is required to have $-CF(CF_3)-$ or $-CF_2\text{-}CF_2-$ directly bonded to $-C(=O)\text{-}F$.

$$CF_2=CF\text{-}O\text{-}R^{f21}\text{-}C(=O)\text{-}F \qquad \text{Formula (2A-2-11)}$$

$$CF_2=CF\text{-}O\text{-}R^{f211}\text{-}CF=CF_2 \qquad \text{Formula (2A-2-12)}$$

$R^{f21}$ in the formula (2A-2-11) is synonymous with $R^{f21}$ in the formula (1A-2-1).

$R^{f211}$ in the formula (2A-2-12) is a perfluoroalkylene group. The number of carbon atoms in the perfluoroalkylene group is preferably from 1 to 8, particularly preferably from 1 to 4. The perfluoroalkylene group may be linear or branched.

As specific examples of the compound represented by the formula (2A-2-11), $CF_2=CF\text{-}O\text{-}CF_2CF_2\text{-}C(=O)\text{-}F$, $CF_2=CF\text{-}O\text{-}CF_2CF_2CF_2\text{-}C(=O)\text{-}F$, $CF_2=CF\text{-}O\text{-}CF_2CF_2CF_2CF_2\text{-}C(=O)\text{-}F$, $CF_2=CF\text{-}O\text{-}CF_2CF_2CF_2CF_2CF_2\text{-}C(=O)\text{-}F$, $CF_2=CF\text{-}O\text{-}CF(CF_3)\text{-}CF_2\text{-}CF_2\text{-}C(=O)\text{-}F$ and $CF_2=CF\text{-}O\text{-}CF_2CF(CF_3)\text{-}CF_2\text{-}C(=O)\text{-}F$ may be mentioned.

As specific examples of the compound represented by the formula (2A-2-12), $CF_2=CF\text{-}O\text{-}CF_2CF=CF_2$, $CF_2=CF\text{-}$

O-CF$_2$CF$_2$-CF=CF$_2$,     CF$_2$=CF-O-CF$_2$CF$_2$CF$_2$-CF=CF$_2$,     CF$_2$=CF-O-CF$_2$CF$_2$CF$_2$CF$_2$-CF=CF$_2$,     CF$_2$=CF-O-CF(CF$_3$)-CF$_2$-CF=CF$_2$, CF$_2$=CF-O-CF$_2$CF(CF$_3$)-CF=CF$_2$, etc., may be mentioned.

A compound represented by the formula (2A-2-11) can be converted to a vinyl ether carboxylic acid ester by a known method such as one disclosed in JP-A- 62-51943. Specifically, there may be a method of reacting an alcohol, or a method of hydrolyzing to a vinyl ether carboxylic acid, and further subjected to an esterification reaction.

In the production method in the present invention, when compound 1A-2-2 is used, a compound represented by the following formula (2A-2-21) or a compound represented by the following formula (2A-2-22) is obtainable. However, in order to obtain the compound represented by the formula (2A-2-22), R$^{f23}$ in the above formula (1A-2-2) is required to have -CF(CF$_3$)- or -CF$_2$-CF$_2$- directly bonded to -C(=O)-F.

$$CF_2=CF-O-(R^{f22}O)_{m2}-R^{f23}-C(=O)-F \qquad \text{Formula (2A-2-21)}$$

$$CF_2=CF-O-(R^{f22}O)_{m2}-R^{f231}-CF=CF_2 \qquad \text{Formula (2A-2-22)}$$

In the formula (2A-2-21) and the formula (2A-2-22), R$^{f22}$ and m2 are, respectively, synonymous with R$^{f22}$ and m2 in the formula (1A-2-2).

R$^{f23}$ in the formula (2A-2-21) is synonymous with R$^{f23}$ in the formula (1A-2-2).

R$^{f231}$ in the formula (2A-2-22) is a perfluoroalkylene group. The number of carbon atoms in the perfluoroalkylene group is preferably from 1 to 6, particularly preferably from 1 to 3. The perfluoroalkylene group may be linear or branched.

As specific examples of the compound represented by the formula (2A-2-22), CF$_2$=CF-O-CF$_2$CF(CF$_3$)O-CF$_2$CF$_2$-C(=O)-F, CF$_2$=CF-O-CF$_2$CF$_2$CF$_2$O-CF$_2$CF$_2$-C(=O)-F and CF$_2$=CF-O-CF$_2$CF$_2$O-CF$_2$CF$_2$-C(=O)-F may be mentioned.

As specific examples of the compound represented by the formula (2A-2-23), CF$_2$=CF-O-CF$_2$CF(CF$_3$)O-CF$_2$-CF=CF$_2$, CF$_2$=CF-O-CF$_2$CF$_2$CF$_2$O-CF$_2$-CF=CF$_2$ and CF$_2$=CF-O-CF$_2$CF$_2$O-CF$_2$-CF=CF$_2$ may be mentioned.

In the production method in the present invention, when compound 1A-3 is used, the compound represented by the following formula (2A-3) is obtainable.

$$CF_2=CF-O-Q^{a3}(-SO_2F)_q \qquad \text{Formula (2A-3)}$$

Q$^{a3}$ and q in the formula (2A-3) are synonymous with Q$^{a3}$ and q in the formula (1A-3), respectively.

In the production method in the present invention, when compound 1A-3-1 is used, the compound represented by the following formula (2A-3-1) is obtainable.

$$CF_2=CF-(OCF_2CFZ^{a1})_{m3}-O_{p3}-(CF_2)_{n3}-SO_2F \qquad \text{Formula (2A-3-1)}$$

Z$^{a1}$, m3, p3 and n3 in the formula (2A-3-1) are synonymous with Z$^{a1}$, m3, p3 and n3 in the formula (1A-3-1), respectively.

As specific examples of the compound represented by the formula (2A-3-1), CF$_2$=CF-OCF$_2$CF$_2$-SO$_2$F, CF$_2$=CF-OCF$_2$CF$_2$CF$_2$-SO$_2$F, CF$_2$=CF-OCF$_2$CF$_2$CF$_2$CF$_2$-SO$_2$F, CF$_2$=CF-OCF$_2$CF$_2$-OCF$_2$CF -SO$_2$F and CF$_2$=CF-OCF$_2$CF(CF$_3$)-OCF$_2$CF$_2$-SO$_2$F may be mentioned.

In the production method in the present invention, when compound 1A-3-2 is used, the compound represented by the following formula (2A-3-2) is obtainable.

$$CF_2=CF-O-(CF_2)_{n4}-(O)_{p4}-C(Z^{a2})(-Q^{a31}-SO_2F)(-Q^{a32}-SO_2F) \qquad \text{Formula (2A-3-2)}$$

n4, p4, Z$^{a2}$, Q$^{a31}$ and Q$^{a32}$ in the formula (2A-3-2) are synonymous with n4, p4, Z$^{a2}$, Q$^{a31}$ and Q$^{a32}$ in the formula (1A-3-2), respectively.

As specific examples of the compound represented by the formula (2A-3-2), CF$_2$=CF-O-CF$_2$-CF(-OCF$_2$CF$_2$-SO$_2$F)(-CF$_2$CF$_2$-SO$_2$F), CF$_2$=CF-O-CF$_2$-CF$_2$CF$_2$OCF(CF$_2$-SO$_2$F)(CF$_2$-SO$_2$F) and CF$_2$=CF-O-CF$_2$-CF(-OCF$_2$CF$_2$-SO$_2$F)(-CF$_2$-OCF$_2$CF$_2$-SO$_2$F) may be mentioned.

In a case where compound 2 has a sulfonyl fluoride group, compound 2 is suitably used as a monomer component for the production of a sulfonic acid group-containing fluorinated polymer. Here, the sulfonic acid group-containing fluorinated polymer is suitably used for the production of an electrolyte membrane.

<Byproducts>

Byproducts include, but are not limited to, compounds (hereinafter referred to also as "specified byproducts") having a hydrogen fluoride added to the vinyl ether group of compound 2. The specified byproducts may sometimes be difficult to separate them from compound 2, or the separation process may sometimes be complicated.

Against such problems, according to the production method in the present invention, it is possible to suppress the formation of byproducts such as specified byproducts and to obtain high-purity compound 2, whereby the separation process, etc. can be simplified.

For example, in a case where compound 1A is used in the method for producing a fluorovinyl ether compound of the present invention, a compound represented by the following formula (3A) may be formed as a specified byproduct.

$$CF_3\text{-CHF-O-R}^f \qquad \text{Formula (3A)}$$

$R^f$ in the formula (3A) is synonymous with $R^f$ in the formula (1A).

EXAMPLES

In the following, the present invention will be described in detail with reference to Examples. Ex. 1-2 to Ex. 1-7, Ex. 2-2 to Ex. 2-7, Ex. 3-2 to Ex. 3-4, Ex. 4-2 to Ex. 4-4, Ex. 5-2 to Ex. 5-5, and Ex. 6-2 to Ex. 6-6 are Examples of the present invention, and Ex. 1-1, Ex. 2-1, Ex. 3-1, Ex. 4-1, Ex. 5-1, and Ex. 6-1 are Comparative Examples. However, the present invention is not limited to these Examples.

All Examples and Comparative Examples were carried out at ambient pressure.

[Specific surface area]

The specific surface area of the oxide was obtained by analyzing the results measured by the gas adsorption method (using nitrogen gas) by using "3Flex" manufactured by Micromeritics Instrument Corporation, by the BET method.

[Crystallinity]

The presence or absence of crystallinity of the oxide was determined based on the diffraction pattern measured by "Smart Lab" manufactured by Rigaku Corporation.

The oxide was considered to be crystalline if it contained diffraction peaks corresponding to the crystalline structure and to be non-crystalline if it did not contain diffraction peaks corresponding to the crystalline structure.

[Amount of byproducts formed relative to the amount of fluorovinyl ether compound formed]

With respect to the product gas collected from the outlet of the reactor after 5 hours from the initiation of the reaction, the gas composition analysis was conducted using a gas chromatograph under the following conditions, and from the area of the peak in the analysis results, the amount of byproducts formed relative to the fluorovinyl ether compound was obtained by the following formula. The smaller the calculated value is, the more the generation of byproducts is suppressed.

Here, the byproducts are a group of compounds that are difficult to separate from the fluorovinyl ether compound, and the total value of byproduct peaks, excluding raw materials whose retention time in the gas chromatograph analysis value is detected less than 20 minutes after the fluorovinyl ether compound, was adopted as the amount of byproducts formed.

Amount of byproducts formed relative to the amount of fluorovinyl ether compound formed = (Percentage of the area of all byproducts except raw materials in the total area of the product gas peak [%]) / (Percentage of the area of fluorovinyl ether compound in the total area of the product gas peak [%])

<Analysis conditions for gas chromatograph>

The gas composition analysis of the product gas was conducted by installing on "6850 gas chromatograph" manufactured by Agilent, as a capillary column, "Rtx-200" manufactured by Restek Corporation (inner diameter 0.25 mm, length 60 m, film thickness 1.00 $\mu$m), and after holding a carrier gas: helium, inlet temperature: 240°C, gas linear velocity: 22.6 cm/s, column temperature: 40°C for 10 minutes, raising the temperature to 240°C at 10°C/min and holding it for 10 minutes, after that, it was detected by the FID detector.

[Conversion ratio]

After 5 hours from the initiation of the reaction, the product gas collected from the outlet of the reactor was collected in a stainless steel cylinder cooled by liquid nitrogen, and the obtained liquid was subjected to the composition analysis by gas chromatograph, whereby the conversion ratio of the raw material was obtained as follows, and the conversion ratio was evaluated according to the following evaluation standards.

Conversion ratio of raw material (%) = {1-(raw material concentration at reactor

outlet (g/g)) / (raw material concentration at reactor inlet (g/g))} × 100

A: Conversion ratio of raw material is at least 70%.
B: Conversion ratio of raw material is at least 50% and less than 70%.
C: Conversion ratio of raw material is at least 30% and less than 50%.
D: Conversion ratio of raw material is at least 10% and less than 30%.
E: Conversion ratio of raw material is less than 10.

[Ex. 1-1]

Into one side (length 700 mm) of a U-shaped reaction tube made of SUS316 and having an inner diameter of 21.4 mm and a total length of 1,550 mm, a stainless steel perforated plate was put at a position of 5 cm from the bottom, and thereon, 53 mL of glass beads 1 (specific surface area: 0.5 $m^2$/g, crystallinity: none, aluminosilicate containing sodium) were packed, and then nitrogen was introduced at 330°C to dry the packed material. The amount of nitrogen introduced was 150 NmL/min. Then, at 330°C, by letting a mixed gas of nitrogen/raw material=93.4/6.6 (mol/mol) flow for a contact time of 10.7 seconds (conducted by a fluidized bed method), a vinyl ether compound was obtained.
Here, the contact time is a value obtained by dividing the oxide packing height by the superficial gas velocity of the mixed gas consisting of the raw material and nitrogen, and the superficial gas velocity was obtained by dividing the mixed gas flow rate at the reaction temperature and reaction pressure by the cross-sectional area of the reaction tube. The same applies in the following [Ex. 1-2] to [Ex. 6-6].
The amount of byproducts formed relative to the amount of the fluorovinyl ether compound formed was 0.10 [Area%/Area%], Further, the evaluation result of the conversion ratio of the raw material was C.
Here, a compound represented by the formula (1A-1-11) was used as the raw material. Further, the obtained fluorovinyl ether compound was a compound represented by the formula (2A-1-11).

$$F-C(=O)-CF(CF_3)-O-CF_2CF_2CF_3 \qquad \text{Formula (1A-1-11)}$$

$$CF_2=CF-O-CF_2CF_2CF_3 \qquad \text{Formula (2A-1-11)}$$

[Ex. 1-2]

Into a U-shaped reaction tube made of SUS316 and having an inner diameter of 21.4 mm, 53 mL of glass beads 2 (specific surface area: 4.1 $m^2$/g, crystallinity: none) were packed, and nitrogen was introduced at 300°C to dry the packed material. The amount of nitrogen introduced was 150 NmL/min. Then, at 300°C, by letting a mixed gas of nitrogen/raw material=90/10 (mol/mol) flow for a contact time of 16.7 seconds (conducted by a fluidized bed method), the fluorovinyl ether compound represented by the above formula (2A-1-11) was obtained. Here, the compound represented by the above formula (1A-1-11) was used as the raw material.
Here, glass beads 2 were produced by applying surface roughening treatment with acid gas to glass beads 1.
The amount of byproducts formed relative to the amount of the fluorovinyl ether compound formed was 0.05 [Area%/Area%], and thus, as compared to Ex. 1-1 using the same raw material, the generation of byproducts was sufficiently suppressed.
Further, the evaluation result of the conversion ratio of the raw material was A.

[Ex. 1-3]

Into a U-shaped reaction tube made of SUS316 and having an inner diameter of 21.4 mm, 53 mL of $Na_2SiO_3$ (specific surface area: 2.5 $m^2$/g, crystallinity: yes) having surface roughening treatment with acid gas applied, was packed, and nitrogen was introduced at 290°C to dry the packed material. The amount of nitrogen introduced was 150 NmL/min. Then, at 290°C, by letting a mixed gas of nitrogen/raw material=90/10 (mol/mol) flow for a contact time of 16.7 seconds (conducted by a fixed bed), the fluorovinyl ether compound represented by the above formula (2A-1-11) was obtained. Here, the compound represented by the above formula (1A-1-11) was used as the raw material.

The amount of byproducts formed relative to the amount of the fluorovinyl ether compound formed was 0.06 [Area%/Area%], and thus, as compared to Ex. 1-1 using the same raw material, the generation of byproducts was sufficiently suppressed. Further, the evaluation result of the conversion ratio of the raw material was B.

[Ex. 1-4]

Into a U-shaped reaction tube made of SUS316 and having an inner diameter of 21.4 mm, 53 mL of $Na_2ZrO_3$ (specific surface area: 1.8 $m^2/g$, crystallinity: yes) having surface roughening treatment with acid gas applied, was packed, and nitrogen was introduced at 290°C to dry the packed material. The amount of nitrogen introduced was 150 NmL/min. Then, at 291°C, by letting a mixed gas of nitrogen/raw material=90/10 (mol/mol) flow for a contact time of 16.7 seconds (conducted in a fixed bed), the fluorovinyl ether compound represented by the above formula (2A-1-11) was obtained. Here, the compound represented by the above formula (1A-1-11) was used as the raw material.
The amount of byproducts formed relative to the amount of the fluorovinyl ether compound formed was 0.04 [Area%/Area%], and thus, as compared to Ex. 1-1 using the same raw material, the generation of byproducts was sufficiently suppressed. Further, the evaluation result of the conversion ratio of the raw material was B.

[Ex. 1-5]

Into a U-shaped reaction tube made of SUS316 and having an inner diameter of 21.4 mm, 53 mL of alumina adsorbent 1 containing Na (one having Axsorb AB manufactured by Nippon Light Metal Company, Ltd. heat-treated at 600°C for 10 hours under air atmosphere, specific surface area: 177 $m^2/g$, crystallinity: yes) was packed, and nitrogen was introduced at 280°C to dry the packed material. The amount of nitrogen introduced was 150 NmL/min. Then, at 280°C, by letting a mixed gas of nitrogen/raw material=90/10 (mol/mol) flow for a contact time of 16.7 seconds (conducted in a fixed bed), the fluorovinyl ether compound represented by the above formula (2A-1-11) was obtained. Here, the compound represented by the above formula (1A-1-11) was used as the raw material.
The amount of byproducts formed relative to the amount of the fluorovinyl ether compound formed was 0.05 [Area%/Area%], and thus, as compared to Ex. 1-1 using the same raw material, the generation of byproducts was sufficiently suppressed. Further, the evaluation result of the conversion ratio of the raw material was B.

[Ex. 1-6]

Into a U-shaped reaction tube made of SUS316 and having an inner diameter of 21.4 mm, 53 mL of alumina adsorbent 2 containing Na (selexsorb COS manufactured by BASF, specific surface area: 150 $m^2/g$, crystallinity: yes) was packed, and nitrogen was introduced at 280°C to dry the packed material. The amount of nitrogen introduced was 150 NmL/min. Then, at 280°C, by letting a mixed gas of nitrogen/raw material=90/10 (mol/mol) flow for a contact time of 16.7 seconds (conducted by a fixed bed method), the fluorovinyl ether compound represented by the above formula (2A-1-11) was obtained. Here, the compound represented by the above formula (1A-1-11) was used as the raw material.
The amount of byproducts formed relative to the amount of the fluorovinyl ether compound formed was 0.05 [Area%/Area%], and thus, as compared to Ex. 1-1 using the same raw material, the generation of byproduct was sufficiently suppressed. Further, the evaluation result of the conversion ratio of the raw material was C.

[Ex. 1-7]

Into a U-shaped reaction tube made of SUS316 and having an inner diameter of 21.4 mm, 53 mL of Zeolite 1 ("Zeolum A-3, Type 585, 20-32mesh" manufactured by TOSOH CORPORATION, specific surface area: 28 $m^2/g$, crystallinity: yes, aluminosilicate containing potassium) was packed, and nitrogen was introduced at 252°C to dry the packed material. The amount of nitrogen introduced was 150 NmL/min. Then, at 252°C, by letting a mixed gas of nitrogen/raw material=90/10 (mol/mol) flow for a contact time of 16.7 seconds (conducted by a fixed bed method), the fluorovinyl ether compound represented by the above formula (2A-1-11) was obtained. Here, the compound represented by the above formula (1A-1-11) was used as the raw material.
The amount of byproducts formed relative to the amount of the fluorovinyl ether compound formed was 0.03 [Area%/Area%], and thus, as compared to Ex. 1-1 using the same raw material, the generation of byproducts was sufficiently suppressed. Further, the evaluation result of the conversion ratio of the raw material was A.

[Ex. 2-1]

Into a U-shaped reaction tube made of SUS316 and having an inner diameter of 21.4 mm, 106 mL of glass beads 1 (specific surface area: 0.5 $m^2/g$, crystallinity: none, aluminosilicate containing sodium) were packed, and nitrogen was introduced at 252°C to dry the packed material. The amount of nitrogen introduced was 150 NmL/min. Then, at 252°C, by letting a mixed gas having water added to nitrogen/raw material=90/10 (mol/mol) at water/raw material=1/2 (mol/mol) flow for a contact time of 21.4 seconds (conducted by a fluidized bed method), a fluorovinyl ether compound was obtained.

The amount of byproducts formed relative to the amount of the fluorovinyl ether compound formed was 0.21 [Area%/Area%], Further, the evaluation result of the conversion ratio of the raw material was D.

Here, a compound represented by the formula (1A-2-11) was used as the raw material. Further, the obtained fluorovinyl ether compound was a compound represented by the formula (2A-2-111).

$$F\text{-}C(=O)\text{-}CF(CF_3)\text{-}O\text{-}CF_2CF_2CF_2\text{-}C(=O)\text{-}F \qquad \text{Formula (1A-2-11)}$$

$$CF_2=CF\text{-}O\text{-}CF_2CF_2CF_2\text{-}C(=O)\text{-}F \qquad \text{Formula (2A-2-111)}$$

[Ex. 2-2]

Into a U-shaped reaction tube made of SUS316 and having an inner diameter of 21.4 mm, 53 mL of glass beads 2 (specific surface area: 4.1 $m^2/g$, crystallinity: none) were packed, and nitrogen was introduced at 251°C to dry the packed material. The amount of nitrogen introduced was 150 NmL/min. Then, at 251°C, by letting a mixed gas having water added to nitrogen/raw material=90/10 (mol/mol) at water/raw material=1/10 (mol/mol) flow for a contact time of 10.7 seconds (conducted by a fluidized bed method), the fluorovinyl ether compound represented by the above formula (2A-2-111) was obtained. Here, the compound represented by the above formula (1A-2-11) was used as the raw material.

The amount of byproducts formed relative to the amount of the fluorovinyl ether compound formed was 0.06 [Area%/Area%], and thus, as compared to Ex. 2-1 using the same raw material, the generation of byproducts was sufficiently suppressed. Further, the evaluation result of the conversion ratio of the raw material was C.

[Ex. 2-3]

Into a U-shaped reaction tube made of SUS316 and having an inner diameter of 21.4 mm, 53 mL of glass beads 3 (specific surface area: 13.0 $m^2/g$, crystallinity: none) were packed, and nitrogen was introduced at 250°C to dry the packed material. The amount of nitrogen introduced was 150 NmL/min. Then, at 252°C, by letting a mixed gas having water added to nitrogen/raw material=90/10 (mol/mol) at water/raw material=1/10 (mol/mol) flow for a contact time of 10.7 seconds (conducted by a fluidized bed method), the fluorovinyl ether compound represented by the above formula (2A-2-111) was obtained. Here, the compound represented by the above formula (1A-2-11) was used as the raw material.

Here, glass beads 3 were produced by applying surface roughening treatment with acid gas to glass beads 1.

The amount of byproducts formed relative to the amount of the fluorovinyl ether compound formed was 0.08 [Area%/Area%], and thus, as compared to Ex. 2-1 using the same raw material, the generation of byproducts was sufficiently suppressed. Further, the evaluation result of the conversion ratio of the raw material was D.

[Ex. 2-4]

Into a U-shaped reaction tube made of SUS316 and having an inner diameter of 21.4 mm, 53 mL of $Na_2SiO_3$ (specific surface area: 2.5 $m^2/g$, crystallinity: yes) having surface roughening treatment with acid gas applied, was packed, and nitrogen was introduced at 250°C to dry the packed material. The amount of nitrogen introduced was 150 NmL/min. Then, at 252°C, by letting a mixed gas having water added to nitrogen/raw material=90/10 (mol/mol) at water/raw material=1/10 (mol/mol) flow for a contact time of 10.7 seconds (conducted by a fixed bed method), the fluorovinyl ether compound represented by the above formula (2A-2-111) was obtained. Here, the compound represented by the above formula (1A-2-11) was used as the raw material.

The amount of byproducts formed relative to the amount of the fluorovinyl ether compound formed was 0.10 [Area%/Area%], and thus, as compared to Ex. 2-1 using the same raw material, the generation of byproducts was sufficiently suppressed. Further, the evaluation result of the conversion ratio of the raw material was C.

[Ex. 2-5]

Into a U-shaped reaction tube made of SUS316 and having an inner diameter of 21.4 mm, 53 mL of alumina adsorbent 1 containing Na was packed, and nitrogen was introduced at 250°C to dry the packed material. The amount of nitrogen introduced was 150 NmL/min. Then, at 250°C, by letting a mixed gas having water added to nitrogen/raw material=90/10 (mol/mol) at water/raw material 1/10 (mol/mol) flow for a contact time of 10.7 seconds (conducted by a fixed bed method), the fluorovinyl ether compound represented by the above formula (2A-2-111) was obtained. Here, the compound represented by the above formula (1A-2-11) was used as the raw material.

The amount of byproducts formed relative to the amount of the fluorovinyl ether compound formed was 0.09 [Area%/Area%], and thus, as compared to Ex. 2-1 using the same raw material, the generation of byproducts was sufficiently suppressed. Further, the evaluation result of the conversion ratio of the raw material was B.

[Ex. 2-6]

Into a U-shaped reaction tube made of SUS316 and having an inner diameter of 21.4 mm, 53 mL of Zeolite 2 ("Zeolum A-4, 14-20mesh" manufactured by TOSOH CORPORATION, specific surface area: 29 $m^2$/g, crystallinity: yes, aluminosilicate containing sodium) was packed, and nitrogen was introduced at 252°C to dry the packed material. The amount of nitrogen introduced was 150 NmL/min. Then, at 251°C, by letting a mixed gas having water added to nitrogen/raw material=90/10 (mol/mol) at water/raw material=1/10 (mol/mol) flow for a contact time of 10.7 seconds (conducted by a fixed bed method), the fluorovinyl ether compound represented by the above formula (2A-2-111) was obtained. Here, the compound represented by the above formula (1A-2-11) was used as the raw material.

The amount of byproducts formed relative to the amount of the fluorovinyl ether compound formed was 0.10 [Area%/Area%], and thus, as compared to Ex. 2-1 using the same raw material, the generation of byproducts was sufficiently suppressed. Further, the evaluation result of the conversion ratio of the raw material was C.

[Ex. 3-1]

Into a U-shaped reaction tube made of SUS316 and having an inner diameter of 21.4 mm, 107 mL of glass beads 1 (specific surface area: 0.5 $m^2$/g, crystallinity: none, aluminosilicate containing sodium) were packed, and nitrogen was introduced at 320°C to dry the packed material. The amount of nitrogen introduced was 150 NmL/min. Then, at 320°C, by letting a mixed gas of nitrogen/raw material=94.5/5.6 (mol/mol) flow for a contact time of 10.7 seconds (conducted by a fluidized bed method), a fluorovinyl ether compound was obtained.

The amount of byproducts formed relative to the amount of the fluorovinyl ether compound formed was 1.24 [Area%/Area%], Further, the evaluation result of the conversion ratio of the raw material was B.

Here, the compound represented by the formula (1A-2-12) was used as the raw material. Further, the obtained fluorovinyl ether compound was a compound represented by the formula (2A-2-121).

$$F\text{-}C(=O)\text{-}CF(CF_3)\text{-}O\text{-}CF_2CF_2CF_2CF_2\text{-}C(=O)\text{-}F \qquad \text{Formula (1A-2-12)}$$

$$CF_2=CF\text{-}O\text{-}CF_2CF_2\text{-}CF=CF_2 \qquad \text{Formula (2A-2-121)}$$

[Ex. 3-2]

Into a U-shaped reaction tube made of SUS316 and having an inner diameter of 21.4 mm, 106 mL of glass beads 4 (specific surface area: 24.4 $m^2$/g, crystallinity: none) were packed, and nitrogen was introduced at 323°C to dry the packed material. The amount of nitrogen introduced was 150 NmL/min. Then, at 323°C, by letting a mixed gas of nitrogen/raw material=93.4/6.6 (mol/mol) flow for a contact time of 10.7 seconds (conducted by a fluidized bed method), the fluorovinyl ether compound represented by the above formula (2A-2-121) was obtained. Here, the compound represented by the above formula (1A-2-12) was used as the raw material.

Here, glass beads 4 were produced by applying surface roughening treatment with acid gas, to glass beads 1.

The amount of byproducts formed relative to the amount of the fluorovinyl ether compound formed was 0.02 [Area%/Area%], and thus, as compared to Ex. 3-1 using the same raw material, the generation of byproducts was sufficiently suppressed. Further, the evaluation result of the conversion ratio of the raw material was A.

[Ex. 3-3]

Into a U-shaped reaction tube made of SUS316 and having an inner diameter of 21.4 mm, 106 mL of $Na_2SiO_3$ (specific surface area: 2.5 $m^2$/g, crystallinity: yes) having surface roughening treatment with acid gas applied, was packed, and nitrogen was introduced at 320°C to dry the packed material. The amount of nitrogen introduced was 150 NmL/min. Then, at 320°C, by letting a mixed gas of nitrogen/raw material=93.4/6.6 (mol/mol) flow for a contact time of 10.7 seconds (conducted by a fixed bed method), the fluorovinyl ether compound represented by the above formula (2A-2-121) was obtained. Here, the compound represented by the above formula (1A-2-12) was used as the raw material.

The amount of byproducts formed relative to the amount of the fluorovinyl ether compound formed was 0.04 [Area%/Area%], and thus, as compared to Ex. 3-1 using the same raw material, the generation of byproduct was sufficiently suppressed. Further, the evaluation result of the conversion ratio of the raw material was B.

[Ex. 3-4]

Into a U-shaped reaction tube made of SUS316 and having an inner diameter of 21.4 mm, 106 mL of alumina adsorbent 1 containing Na, was packed, and nitrogen was introduced at 290°C to dry the packed material. The amount of nitrogen introduced was 150 NmL/min. Then, at 291°C, by letting a mixed gas of nitrogen/raw material=93.4/6.6 (mol/mol) flow for a contact time of 21.4 seconds (conducted by a fixed bed method), the fluorovinyl ether compound represented by the above formula (2A-2-121) was obtained. Here, the compound represented by the above formula (1A-2-12) was used as the raw material.

The amount of byproducts formed relative to the amount of the fluorovinyl ether compound formed was 0.06 [Area%/Area%], and thus, as compared to Ex. 3-1 using the same raw material, the generation of byproducts was sufficiently suppressed. Further, the evaluation result of the conversion ratio of the raw material was B.

[Ex. 4-1]

Into a U-shaped reaction tube made of SUS316 and having an inner diameter of 21.4 mm, 53 mL of glass beads 1 (specific surface area: 0.5 $m^2$/g, crystallinity: none, aluminosilicate containing sodium) were packed, and nitrogen was introduced at 320°C to dry the packed material. The amount of nitrogen introduced was 150 NmL/min. Then, at 320°C, by letting a mixed gas of nitrogen/raw material=95.0/5.0 (mol/mol) flow for a contact time of 10.1 seconds (conducted by a fluidized bed method), the fluoroallyl ether compound was obtained.

The amount of byproducts formed relative to the amount of the fluoroallyl ether compound formed was 0.15 [Area%/Area%], Further, the evaluation result of the conversion ratio of the raw material was D.

Here, the compound represented by the formula (1A-2-21) was used as the raw material. Further, the fluoroallyl ether compound obtained was the compound represented by the formula (2A-2-221).

$$F-C(=O)-CF(CF_3)-OCF_2CF(CF_3)-OCF_2CF_2CF_2-C(=O)-F \qquad \text{Formula (1A-2-21)}$$

$$CF_2=CF-OCF_2CF(CF_3)-OCF_2CF=CF_2 \qquad \text{Formula (2A-2-221)}$$

[Ex. 4-2]

Into a U-shaped reaction tube made of SUS316 and having an inner diameter of 21.4 mm, 106 mL of glass beads 5 (specific surface area: 5.1 $m^2$/g, crystallinity: none) were packed, and nitrogen was introduced at 320°C to dry the packed material. The amount of nitrogen introduced was 150 NmL/min. Then, at 320°C, by letting a mixed gas of nitrogen/raw material=95.0/5.0 (mol/mol) flow for a contact time of 10.5 seconds (conducted by a fluidized bed method), the fluoroallyl ether compound represented by the above formula (2A-2-221) was obtained. Here, the compound represented by the above formula (1A-2-21) was used as the raw material.

Here, glass beads 5 were produced by applying surface roughening treatment with acid gas, to glass beads 1.

The amount of byproducts formed relative to the amount of the fluoroallyl ether compound formed was 0.06 [Area%/Area%], and thus, as compared to Ex. 4-1 using the same raw material, the generation of byproducts was sufficiently suppressed. Further, the evaluation result of the conversion ratio of the raw material was A.

[Ex. 4-3]

Into a U-shaped reaction tube made of SUS316 and having an inner diameter of 21.4 mm, 106 mL of $Na_2SiO_3$ (specific surface area: 2.5 $m^2$/g, crystallinity: yes) having surface roughening treatment with acid gas applied, were packed, and nitrogen was introduced at 320°C to dry the packed material. The amount of nitrogen introduced was 150 NmL/min. Then, at 300°C, by letting a mixed gas of nitrogen/raw material=95.0/5.0 (mol/mol) flow for a contact time of 10.2 seconds (conducted by a fixed bed method), the fluoroallyl ether compound represented by the above formula (2A-2-221) was obtained. Here, the compound represented by the above formula (1A-2-21) was used as the raw material.

The amount of byproducts formed relative to the amount of the fluoroallyl ether compound formed was 0.05 [Area%/Area%], and thus, as compared to Ex. 4-1 using the same raw material, the generation of byproducts was sufficiently suppressed. Further, the evaluation result of the conversion ratio of the raw material was B.

[Ex. 4-4]

Into a U-shaped reaction tube made of SUS316 and having an inner diameter of 21.4 mm, 106 mL of alumina adsorbent 1 containing Na, was packed, and nitrogen was introduced at 300°C to dry the packed material. The amount of nitrogen introduced was 150 NmL/min. Then, at 280°C, by letting a mixed gas of nitrogen/raw material=95.0/5.0 (mol/mol) flow for a contact time of 10.3 seconds (conducted by a fixed bed method), the fluoroallyl ether compound represented by the above formula (2A-2-221) was obtained. Here, the compound represented by the above formula (1A-2-21) was used as the raw material.

The amount of byproducts formed relative to the amount of the fluoroallyl ether compound formed was 0.03 [Area%/Area%], and thus, as compared to Ex. 4-1 using the same raw material, the generation of byproducts was sufficiently suppressed. Further, the evaluation result of the conversion ratio of the raw material was B.

[Ex. 5-1]

Into a U-shaped reaction tube made of SUS316 and having an inner diameter of 21.4 mm, 53 mL of glass beads 1 (specific surface area: 0.5 $m^2$/g, crystallinity: none, aluminosilicate containing sodium), were packed, and nitrogen was introduced at 330°C to dry the packed material. The amount of nitrogen introduced was 150 NmL/min. Then, at 330°C, by letting a mixed gas of nitrogen/raw material=93.4/6.6 (mol/mol) flow for a contact time of 10.7 seconds (conducted by a fluidized bed method), a fluorovinyl ether compound was obtained.

The amount of byproducts formed relative to the amount of the fluorovinyl ether compound formed was 0.08 [Area%/Area%], Further, the evaluation result of the conversion ratio of the raw material was B.

Here, the compound represented by the formula (1A-3-11) was used as the raw material. Further, the fluorovinyl ether compound obtained was the compound represented by the formula (2A-3-11).

$$F\text{-}C(=O)\text{-}CF(CF_3)\text{-}OCF_2CF(CF_3)\text{-}OCF_2CF_2\text{-}SO_2F \qquad \text{Formula (1A-3-11)}$$

$$CF_2=CF\text{-}OCF_2CF(CF_3)\text{-}OCF_2CF_2\text{-}SO_2F \qquad \text{Formula (2A-3-11)}$$

[Ex. 5-2]

Into a U-shaped reaction tube made of SUS316 and having an inner diameter of 21.4 mm, 106 mL of glass beads 5 (specific surface area: 5.1 $m^2$/g, crystallinity: none), were packed, and nitrogen was introduced at 331°C to dry the packed material. The amount of nitrogen introduced was 150 NmL/min. Then, at 331°C, by letting a mixed gas of nitrogen/raw material=93.4/6.6 (mol/mol) flow for a contact time of 21.4 seconds (conducted by a fluidized bed method), the fluorovinyl ether compound represented by the above formula (2A-3-11) was obtained. Here, the compound represented by the above formula (1A-3-11) was used as the raw material.

Here, glass beads 5 were produced by applying surface roughening treatment with acid gas, to glass beads 1.

The amount of byproducts formed relative to the amount of the fluorovinyl ether compound formed was 0.01 [Area%/Area%], and thus, as compared to Ex. 5-1 using the same raw material, the generation of byproducts was sufficiently suppressed. Further, the evaluation result of the conversion ratio of the raw material was A.

[Ex. 5-3]

Into a U-shaped reaction tube made of SUS316 and having an inner diameter of 21.4 mm, 106 mL of $Na_2SiO_3$ (specific surface area: 2.5 $m^2$/g, crystallinity: yes) having surface roughening treatment with acid gas applied, was packed, and nitrogen was introduced at 310°C to dry the packed material. The amount of nitrogen introduced was 150 NmL/min. Then, at 310°C, by letting a mixed gas of nitrogen/raw material=93.4/6.6 (mol/mol) flow for a contact time of 21.4 seconds (conducted by a fixed bed method), the fluorovinyl ether compound represented by the above formula (2A-3-11) was obtained. Here, the compound represented by the above formula (1A-3-11) was used as the raw material.

The amount of byproducts formed relative to the amount of the fluorovinyl ether compound formed was 0.05 [Area%/Area%], and thus, as compared to Ex. 5-1 using the same raw material, the generation of byproduct was sufficiently suppressed. Further, the evaluation result of the conversion ratio of the raw material was B.

[Ex. 5-4]

Into a U-shaped reaction tube made of SUS316 and having an inner diameter of 21.4 mm, 106 mL of alumina adsorbent 1 containing Na, was packed, and nitrogen was introduced at 281°C to dry the packed material. The amount of nitrogen introduced was 150 NmL/min. Then, at 280°C, by letting a mixed gas of nitrogen/raw material=93.4/6.6 (mol/mol) flow for a contact time of 21.4 seconds (conducted by a fixed bed method),the fluorovinyl ether compound represented by the above formula (2A-3-11) was obtained. Here, the compound represented by the above formula (1A-3-11) was used as the raw material.

The amount of byproducts formed relative to the amount of the fluorovinyl ether compound formed was 0.04 [Area%/Area%], and thus, as compared to Ex. 5-1 using the same raw material, the generation of byproducts was sufficiently suppressed. Further, the evaluation result of the conversion ratio of the raw material was B.

[Ex. 5-5]

Into a U-shaped reaction tube made of SUS316 and having an inner diameter of 21.4 mm, 106 mL of Zeolite 3 ("HSZ-300 Type 320NAD1C" manufactured by TOSOH CORPORATION, specific surface area: 640 $m^2$/g, crystallinity: yes, aluminosilicate containing sodium), was packed, and nitrogen was introduced at 252°C to dry the packed material. The amount of nitrogen introduced was 150 NmL/min. Then, at 252°C, by letting a mixed gas of nitrogen/raw material=93.4/6.6 (mol/mol) flow for a contact time of 21.4 seconds (conducted by a fixed bed method), the fluorovinyl ether compound represented by the above formula (2A-3-11) was obtained. Here, the compound represented by the above formula (1A-3-11) was used as the raw material.

The amount of byproducts formed relative to the amount of the fluorovinyl ether compound formed was 0.07 [Area%/Area%], and thus, as compared to Ex. 5-1 using the same raw material, the generation of byproducts was sufficiently suppressed. Further, the evaluation result of the conversion ratio of the raw material was D.

[Ex. 6-1]

Into a U-shaped reaction tube made of SUS316 and having an inner diameter of 21.4 mm, 53 mL of glass beads 1 (specific surface area: 0.5 $m^2$/g, crystallinity: none, aluminosilicate containing sodium), were packed, and nitrogen was introduced at 330°C to dry the packed material. The amount of nitrogen introduced was 150 NmL/min. Then, at 330°C, by letting a mixed gas of nitrogen/raw material=93.4/6.6 (mol/mol) flow for a contact time of 7.5 seconds (conducted by a fluidized bed method), the fluorovinyl ether compound was obtained.

The amount of byproducts formed relative to the amount of the fluorovinyl ether compound formed was 0.50 [Area%/Area%], Further, the evaluation result of the conversion ratio of the raw material was D.

Here, the compound represented by the formula (1A-3-21) was used as the raw material. Further, the obtained fluorovinyl ether compound was a compound represented by the formula (2A-3-21).

$$F-C(=O)-CF(CF_3)-O-CF_2-CF(-OCF_2CF_2-SO_2F)(-CF_2-OCF_2CF_2-SO_2F) \qquad \text{Formula (1A-3-21)}$$

$$CF_2=CF-O-CF_2-CF(-OCF_2CF_2-SO_2F)(-CF_2-OCF_2CF_2-SO_2F) \qquad \text{Formula (2A-3-21)}$$

[Ex. 6-2]

Into a U-shaped reaction tube made of SUS316 and having an inner diameter of 21.4 mm, 53 mL of glass beads 6 (specific surface area: 12.3 $m^2$/g, crystallinity: none) were packed, and nitrogen was introduced at 331°C to dry the packed material. The amount of nitrogen introduced was 150 NmL/min. Then, at 331°C, by letting a mixed gas of nitrogen/raw material=93.4/6.6 (mol/mol) flow for a contact time of 7.5 seconds (conducted by a fluidized bed method), the fluorovinyl ether compound represented by the above formula (2A-3-21) was obtained. Here, the compound represented by the above formula (1A-3-21) was used as the raw material.

Here, glass beads 6 were produced by applying surface roughening treatment with acid gas to glass beads 1.

The amount of byproducts formed relative to the amount of the fluorovinyl ether compound formed was 0.02 [Area%/Area%], and thus, as compared to Ex. 6-1 using the same raw material, the generation of byproducts was sufficiently suppressed. Further, the evaluation result of the conversion ratio of the raw material was B.

[Ex. 6-3]

Into a U-shaped reaction tube made of SUS316 and having an inner diameter of 21.4 mm, 53 mL of glass beads 3 (specific surface area: 13.0 $m^2$/g, crystallinity: none) were packed, and nitrogen was introduced at 333°C to dry the packed material. The amount of nitrogen introduced was 150 NmL/min. Then, at 333°C, by letting a mixed gas of nitrogen/raw material=93.4/6.6 (mol/mol) flow for a contact time of 7.5 seconds (conducted by a fluidized bed method), the fluorovinyl ether compound represented by the above formula (2A-3-21) was obtained. Here, the compound represented by the above formula (1A-3-21) was used as the raw material.

The amount of byproducts formed relative to the amount of the fluorovinyl ether compound formed was 0.03 [Area%/Area%], and thus, as compared to Ex. 6-1 using the same raw material, the generation of byproducts was sufficiently suppressed. Further, the evaluation result of the conversion ratio of the raw material was B.

[Ex. 6-4]

Into a U-shaped reaction tube made of SUS316 and having an inner diameter of 21.4 mm, 53 mL of $Na_2SiO_3$ (specific surface area: 2.5 $m^2$/g, crystallinity: yes) having surface roughening treatment with acid gas applied, was packed, and nitrogen was introduced at 320°C to dry the packed material. The amount of nitrogen introduced was 150 NmL/min. Then, at 320°C, by letting a mixed gas of nitrogen/raw material=93.4/6.6 (mol/mol) flow for a contact time of 7.5 seconds (conducted by a fluidized bed method), the fluorovinyl ether compound represented by the above formula (2A-3-21) was obtained. Here, the compound represented by the above formula (1A-3-21) was used as the raw material.

The amount of byproducts formed relative to the amount of the fluorovinyl ether compound formed was 0.10 [Area%/Area%], and thus, as compared to Ex. 6-1 using the same raw material, the generation of byproducts was sufficiently suppressed. Further, the evaluation result of the conversion ratio of the raw material was B.

[Ex. 6-5]

Into a U-shaped reaction tube made of SUS316 and having an inner diameter of 21.4 mm, 53 mL of alumina adsorbent 1 containing Na, was packed, and nitrogen was introduced at 301°C to dry the packed material. The amount of nitrogen introduced was 150 NmL/min. Then, at 300°C, by letting a mixed gas of nitrogen/raw material=93.4/6.6 (mol/mol) flow for a contact time of 7.5 seconds (conducted by a fluidized bed method), the fluorovinyl ether compound represented by the above formula (2A-3-21) was obtained. Here, the compound represented by the above formula (1A-3-21) was used as the raw material.

The amount of byproducts formed relative to the amount of the fluorovinyl ether compound formed was 0.05 [Area%/Area%], and thus, as compared to Ex. 6-1 using the same raw material, the generation of byproducts was sufficiently suppressed. Further, the evaluation result of the conversion ratio of the raw material was C.

[Ex. 6-6]

Into a U-shaped reaction tube made of SUS316 and having an inner diameter of 21.4 mm, 53 mL of Zeolite 4 ("HSZ-500 Type 500KODAC" manufactured by TOSOH CORPORATION, specific surface area: 257 $m^2$/g, crystallinity: yes, aluminosilicate containing potassium), was packed, and nitrogen was introduced at 301°C to dry the packed material. The amount of nitrogen introduced was 150 NmL/min. Then, at 301°C, by letting a mixed gas of nitrogen/raw material=93.4/6.6 (mol/mol) flow for a contact time of 7.5 seconds (conducted by a fixed bed method), the fluorovinyl ether compound represented by the above formula (2A-3-21) was obtained. Here, the compound represented by the above formula (1A-3-21) was used as the raw material.

The amount of byproducts formed relative to the amount of the fluorovinyl ether compound formed was less than 0.01 [Area%/Area%], and thus, as compared to Ex. 6-1 using the same raw material, the generation of byproducts was sufficiently suppressed.

The entire disclosure of Japanese Patent Application No. 2020-217715 filed on December 25, 2020 including specification, claims, drawings and summary is incorporated herein by reference in its entirety.

**Claims**

1. A method for producing a fluorovinyl ether compound, which comprises heat-treating a compound having a group represented by the following formula (1) in the presence of an oxide containing at least one element selected from the group consisting of alkali metal elements and alkaline earth metal elements, to obtain a fluorovinyl ether compound having a group represented by the following formula (2), wherein the specific surface area of the oxide before the heat treatment is at least 1.0 $m^2$/g:

$$F-C(=O)-CF(X)-(CF_2)_n-O- \qquad \text{Formula (1)}$$

$$CF_2=CF-O- \qquad \text{Formula (2)}$$

in the formula (1), n is 0 or 1, and when n is 0, X is $CF_3$ and when n is 1, X is F.

2. The method for producing a fluorovinyl ether compound according to Claim 1, wherein the oxide contains an alkali metal element.

3. The method for producing a fluorovinyl ether compound according to any one of Claims 1 and 2, wherein the oxide is an oxide containing at least one element selected from the group consisting of alkali metal elements and alkaline earth metal elements, and another metal element.

4. The method for producing a fluorovinyl ether compound according to any one of Claims 1 to 3, wherein the oxide is a silicate, aluminate, aluminosilicate, borosilicate or aluminoborosilicate containing at least one element selected from the group consisting of alkali metal elements and alkaline earth metal elements.

5. The method for producing a fluorovinyl ether compound according to any one of Claims 1 to 4, wherein the oxide is an amorphous oxide selected from glass, amorphous silica, amorphous alumina and amorphous silica-alumina, or a crystalline oxide selected from crystalline silica, crystalline alumina and crystalline silica-alumina.

6. The method for producing a fluorovinyl ether compound according to any one of Claims 1 to 5, wherein the specific surface area of the oxide before the heat treatment is from 1.0 to 700 $m^2$/g.

7. The method for producing a fluorovinyl ether compound according to any one of Claims 1 to 6, wherein the heat treatment is conducted in the presence of the oxide and another oxide different from the oxide, wherein the amount of the oxide used is from 0.1 to 99 mass% to the total amount of the oxide and another oxide used.

8. The method for producing a fluorovinyl ether compound according to any one of Claims 1 to 7, wherein the compound having the group represented by the above formula (1) is a perfluorinated compound.

9. The method for producing a fluorovinyl ether compound according to any one of Claims 1 to 8, wherein the compound represented by the above formula (1) is a compound represented by the following formula (1A):

$$F-C(=O)-CF(CF_3)-O-R^f \qquad \text{Formula (1A)}$$

in the formula (1A), $R^f$ represents a perfluoroalkyl group which may have a monovalent substituent selected from the group consisting of -C(=O)F, a sulfonyl fluoride group, a nitrile group and a methyl ester group, or a monovalent group having $-CF_2-$ of the perfluoroalkyl group which may have a monovalent substituent, substituted by an etheric oxygen atom.

10. The method for producing a fluorovinyl ether compound according to Claim 9, wherein the compound represented by the above formula (1A) is a compound represented by the following formula (1A-1), a compound represented by the following formula (1A-2) or a compound represented by the following formula (1A-3):

$$F-C(=O)-CF(CF_3)-O-Z^{a1} \qquad \text{Formula (1A-1)}$$

($Z^{a1}$ is a perfluoroalkyl group, or a monovalent group having $-CF_2-$ of the perfluoroalkyl group substituted by an etheric oxygen atom),

$$F-C(=O)-CF(CF_3)-O-Q^{a2}-C(=O)-F \qquad \text{Formula (1A-2)}$$

($Q^{a2}$ is a perfluoroalkylene group, or a divalent group having $-CF_2-$ of the perfluoroalkylene group substituted by an etheric oxygen atom),

$$F-C(=O)-CF(CF_3)-O-Q^{a3}(-SO_2F)_q \qquad \text{Formula (1A-3)}$$

($Q^{a3}$ is a (q+1)-valent perfluorohydrocarbon group or a (q+1)-valent group having $-CF_2-$ of the perfluorohydrocarbon group substituted by an etheric oxygen atom).

11. The method for producing a fluorovinyl ether compound according to any one of Claims 1 to 10, wherein the compound represented by the above formula (2) is a compound represented by the following formula (2A):

$$CF_2=CF-O-R^f \qquad \text{Formula (2A)}$$

($R^f$ is synonymous with $R^f$ in the formula (1A)).

12. The method for producing a fluorovinyl ether compound according to Claim 11, wherein the compound represented by the above formula (2A) is a compound represented by the following formula (2A-1), a compound represented by the following formula (2A-2-1), a compound represented by the following formula (2A-2-2), or a compound represented by the following formula (2A-3):

$$CF_2=CF-O-Z^{a1} \qquad \text{Formula (2A-1)}$$

($Z^{a1}$ in the formula (2A-1) is synonymous with $Z^{a1}$ in the formula (1A-1),

$$CF_2=CF-O-Q^{a2}-C(=O)-F \qquad \text{Formula (2A-2-1)}$$

$$CF_2=CF-O-Q^{a21}-CF=CF_2 \qquad \text{Formula (2A-2-2)}$$

($Q^{a2}$ in the formula (2A-2-1) is synonymous with $Q^{a3}$ in the formula (1A-2), $Q^{a21}$ in the formula (2A-2-2) is a perfluoroalkylene group, or a divalent group having $-CF_2-$ of the perfluoroalkylene group substituted with an etheric oxygen atom),

$$CF_2=CF-O-Q^{a3}(-SO_2F)_q \qquad \text{Formula (2A-3)}$$

($Q^{a3}$ and q in the formula (2A-3) are synonymous with $Q^{a3}$ and q in the formula (1A-3), respectively).

13. The method for producing a fluorovinyl ether compound according to any one of Claims 1 to 12, wherein the oxide is dried prior to the heat treatment.

## INTERNATIONAL SEARCH REPORT

| International application No. |
| --- |
| **PCT/JP2021/047380** |

**A. CLASSIFICATION OF SUBJECT MATTER**

*C07C 41/18*(2006.01)i; *C07C 43/17*(2006.01)i; *C07C 51/58*(2006.01)i; *C07C 59/60*(2006.01)i; *C07C 303/22*(2006.01)i; *C07C 309/82*(2006.01)i; *C07B 61/00*(2006.01)i
FI: C07C41/18; C07C43/17; C07C51/58; C07C59/60; C07C309/82; C07C303/22; C07B61/00 300

According to International Patent Classification (IPC) or to both national classification and IPC

**B. FIELDS SEARCHED**

Minimum documentation searched (classification system followed by classification symbols)
C07C41/18; C07C43/17; C07C51/58; C07C59/60; C07C303/22; C07C309/82; C07B61/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2019-014667 A (AGC INC.) 31 January 2019 (2019-01-31) whole document, in particular, paragraphs [0001], [0006], [0010]-[0012], [0014]-[0019], [0038]-[0041] | 1-13 |
| Y | JP 42-005652 B1 (E.I. DU PONT DE NEMOURS & CO.) 08 March 1967 (1967-03-08) whole document, in particular, p. 1, right column, lines 4-14, p. 1, right column, lines 21-24, p. 2, left column, lines 14, 15, p. 2, left column, lines 24-28, example 10 | 1-13 |
| Y | JP 2004-346014 A (ASAHI GLASS CO., LTD.) 09 December 2004 (2004-12-09) paragraphs [0004], [0005], [0007], [0019]-[0022], [0062], [0063] | 1-13 |
| Y | JP 2002-275106 A (ZAKRYTOE AKTSIONERNOE OBSCHESTVO NAUCHNO-PROIZVODSTVENNOE OBIEDINENIE "PIM-INVEST") 25 September 2002 (2002-09-25) paragraphs [0001], [0013], [0015]-[0017], [0024], [0027]-[0029] | 1-13 |
| Y | WO 2002/026682 A1 (ASAHI GLASS CO., LTD.) 04 April 2002 (2002-04-04) p. 12, line 16 to p. 14, line 7, example 7 | 1-13 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **16 February 2022** | **01 March 2022** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 **Japan** | |
| | Telephone No. |

Form PCT/ISA/210 (second sheet) (January 2015)

**INTERNATIONAL SEARCH REPORT**

International application No.

**PCT/JP2021/047380**

C.    DOCUMENTS CONSIDERED TO BE RELEVANT

| Category* | Citation of document, with indication, where appropriate, of the relevant passages | Relevant to claim No. |
|---|---|---|
| A | WO 2015/029839 A1 (ASAHI GLASS CO., LTD.) 05 March 2015 (2015-03-05) paragraphs [0095], [0107], [0136], [0142] | 1-13 |
| A | JP 06-009474 A (DAIKIN INDUSTRIES, LTD.) 18 January 1994 (1994-01-18) paragraphs [0010], [0018]-[0020], [0023] | 1-13 |
| A | JP 2004-323413 A (ASAHI GLASS CO., LTD.) 18 November 2004 (2004-11-18) paragraphs [0001], [0055]-[0058], [0069], [0070] | 1-13 |
| A | JP 2017-226617 A (DAIKIN INDUSTRIES, LTD.) 28 December 2017 (2017-12-28) paragraphs [0018], [0019], [0063]-[0070], [0102] | 1-13 |

Form PCT/ISA/210 (second sheet) (January 2015)

**INTERNATIONAL SEARCH REPORT**
Information on patent family members

International application No.

**PCT/JP2021/047380**

| Patent document cited in search report | | | Publication date (day/month/year) | Patent family member(s) | | | Publication date (day/month/year) |
|---|---|---|---|---|---|---|---|
| JP | 2019-014667 | A | 31 January 2019 | (Family: none) | | | |
| JP | 42-005652 | B1 | 08 March 1967 | (Family: none) | | | |
| JP | 2004-346014 | A | 09 December 2004 | (Family: none) | | | |
| JP | 2002-275106 | A | 25 September 2002 | (Family: none) | | | |
| WO | 2002/026682 | A1 | 04 April 2002 | (Family: none) | | | |
| WO | 2015/029839 | A1 | 05 March 2015 | US | 2016-0152545 | A1 | |
| | | | | EP | 3040327 | A1 | |
| JP | 06-009474 | A | 18 January 1994 | (Family: none) | | | |
| JP | 2004-323413 | A | 18 November 2004 | (Family: none) | | | |
| JP | 2017-226617 | A | 28 December 2017 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3291843 A
- JP 62051943 A

- JP 2020217715 A

**Non-patent literature cited in the description**

- *J. Am. Chem. Soc,* 1953, vol. 75 (18), 4525-4528